# EUROPEAN PATENT APPLICATION

(11) **EP 2 545 794 A1**
(43) Date of publication of application: **16.01.2013**
(21) Application number: 11753101.2
(22) Date of filing: 31.01.2011
(51) Int. Cl.: A41D 13/00, A41B 9/02, A41B 9/04, A41D 27/26, A61F 5/01

(54) **PROTECTOR**

(30) Priority: 08.03.2010 JP 2010051183
(71) Applicant: Unicharm Corporation, Ehime 799-0111 (JP)
(72) Inventor: MIZUTANI, Katsumi, Kanonji-shi Kagawa 769-1602 (JP); YAMAMOTO, Hiromi, Kanonji-shi Kagawa 769-1602 (JP); HIDE, Kazushi, Tokyo 113-8543 (JP); ICHINOSE, Masafumi, Tokyo 113-8543 (JP)
(74) Representative: Vaughan, Jennifer Ann
(86) International application number: PCT/JP2011/051917
(87) International publication number: WO 2011/111439

(57) **Abstract**

Disclosed is a protector that lightens a shock when a wearer falls, and reduces an unpleasant feeling given to a wearer when worn for a long period of time. Protector 1 is substantially circular in a plan view and is disposed at a position to cover near a femoral-neck portion 21 from a side portion of a wearer. The protector 1 is curved in a thickness direction so that an outer surface 10, comprising a single-layer structure composed of a polymer-foam body, is convex, and an inner surface 20 is concave. An amount of displacement in the thickness direction when a load of 20 N is applied at a central portion of the outer surface 10 when placed on a flat surface is 3 mm - 20 mm.

## Description

### TECHNICAL FIELD

The present invention relates to a protector. More particularly, the present invention relates to a protector that is disposed at a position covering a proximate region to the femoral-neck portion from a lateral side of a wearer.

### BACKGROUND ART

Typically, aged persons exhibit a higher rate of falls than young persons due to deterioration in muscle strength as a result of aging. Furthermore, aged persons exhibit deterioration in bone strength and therefore a fracture of the hand or leg may result from a fall. A serious reduction in walking capacity may result in particular from a fracture to the femur when suffering this type of fracture from a fall. A person may be confined to bed as a result of a fracture to the femur.

In this context, to prevent fracture of the femur resulting from a fall, a protector has been proposed that has a curved shape so that the outer surface side is convex to thereby protect the proximate region to the femoral neck that exhibits a particular tendency to fracture within the femur (For example, reference is made to Patent Documents 1 to 3).

The protector proposed in Patent Document 1 is configured by embedding a reinforcing core configured from a plate member of hard propylene in a polypropylene foam body. The protector proposed in Patent Documents 2 and 3 is configured from an outer layer configured from a polymer-foam body that has resilience or elasticity, and an inner layer that is configured from a polymer-foam body that has flexibility.
Patent Document 1: Japanese Translation of PCT International Publication, Publication No. H10-512016
Patent Document 2: Japanese Unexamined Patent Application, Publication No. 2001-123311
Patent Document 3: Japanese Unexamined Patent Application, Publication No. 2002-30502

### DISCLOSURE OF THE INVENTION

### Problems to be Solved by the Invention

However, the protector proposed in Patent Document 1 does not exhibit sufficient tracking (followability) in relation to bodily movement by a wearer since a plate-shaped reinforcing core is provided that is configured from hard polypropylene. A protector for prevention of a fracture due to a fall must be worn for a long time during the daily life of a wearer, and when a protector that does not exhibit sufficient tracking characteristics is worn for a long time, a wearer may experience an increase in an unpleasant feeling.

On the other hand, the technique proposed in Patent Documents 2 and 3 absorbs a shock on an outer layer and enhances tracking characteristics by the inner layer in relation to the bodily movements of a wearer by configuring the protector with a double-layer structure including an outer layer that exhibits resilience or elasticity and an inner layer that exhibits flexibility. However, the double-layer structure of the protector in the technique proposed in Patent Documents 2 and 3 increases the thickness of the protector. Consequently, the wearer experiences an unpleasant feeling resulting from the increased thickness of the protector.

Therefore, the present invention has the object of providing a protector that reduces an unpleasant feeling given to a wearer during long-term use and enables a reduction in the shock given to the body of a wearer when falling.

### Means for Solving the Problems

The present invention relates to a protector having a substantially circular shape in plan view, and disposed at a position at which a region proximate to a femoral-neck portion is covered from the lateral orientation of a wearer; wherein the protector has a single-layer structure configured from a polymer-foam body; and curves in a thickness direction so that an outer surface is convex and an inner surface is concave; in which a displacement amount in a thickness direction when a 20N force is applied to a central portion of the outer surface when disposed on a flat surface is 3 mm to 20 mm.

Furthermore, the present invention relates to a protector having a substantially circular shape in a plan view, and disposed at a position at which a region proximate to a femoral-neck portion is covered from the lateral orientation of a wearer; wherein the protector has a single-layer structure configured from a polymer-foam body; curves in a thickness direction so that an outer surface is convex and an inner surface is concave to thereby form a space between the inner surface and the skin of a wearer when worn; and deforms into a flat configuration when a 20N force is applied to a central portion of the outer surface.

The protector preferably reduces a force of 9200 N applied from the outer surface in a thickness direction to less than or equal to 3000 N on the inner surface side.

The protector preferably applies a displacement amount in a direction that is orthogonal to the thickness direction when a 6N force from a direction orthogonal to a thickness direction of at least 10 mm.

The density of the polymer-foam body is preferably 0.60 to 1.50 g/cm³.

It is preferred to include shape restoring characteristics when an applied force is released.

The protector preferably further comprises a plurality of holes formed in proximity to the peripheral edge portion in the plan view, piercing in the thickness direction thereof.

An apparent thickness is preferably 10 mm to 25 mm.

The present invention relates to an undergarment configured to contain the protector as described above, the undergarment comprising a hip surrounding portion configured to cover a circumference of a region proximate to the femoral-neck portion of the wearer; a waist opening portion provided on one end of the hip surrounding portion; a pair of leg opening portions provided on the other end of the hip surrounding portion; and a pair of protector-containing portions provided at a position corresponding to both side portions in proximity to the femoral-neck portion of the wearer in the hip surrounding portion.

The present invention relates to an undergarment comprising: a hip surrounding portion configured to cover a circumference of a region proximate to the femoral-neck portion of the wearer; a waist opening portion provided on one end of the hip surrounding portion; and a pair of leg opening portions provided on the other end of the hip surrounding portion; and wherein the protector as described in any configuration above is disposed at a position corresponding to both side portions in proximity to the femoral-neck portion that connects the pelvis and the femur of a wearer in the hip surrounding portion.

### Effects of the Invention

According to the protector of the present invention, an unpleasant feeling given to a wearer during long-term use is reduced and the shock given to the body of a wearer when falling is reduced.

### BRIEF DESCRIPTION OF THE DRAWINGS

FIG. 1 is a front view illustrating a wearer who wears a protector according to the present invention;
FIG. 2 is a perspective view illustrating an embodiment of the protector according to the present invention;
FIG. 3 is a plan view of the protector illustrated in FIG. 2;
FIG. 4 is a sectional view along the line A - A in FIG. 3;
FIG. 5 is a sectional view illustrating a wearer when the protector according to the present invention is attached;
FIG. 6A illustrates the sequence of attaching the protector according to the present invention;
FIG. 6B illustrates the sequence of attaching the protector according to the present invention;
FIG. 6C illustrates the sequence of attaching the protector according to the present invention; and
FIG. 7 is a plan view illustrating a protector according to a second embodiment.

### PREFERRED MODE FOR CARRYING OUT THE INVENTION

The preferred embodiments of the present invention will be described below making reference to the figures.

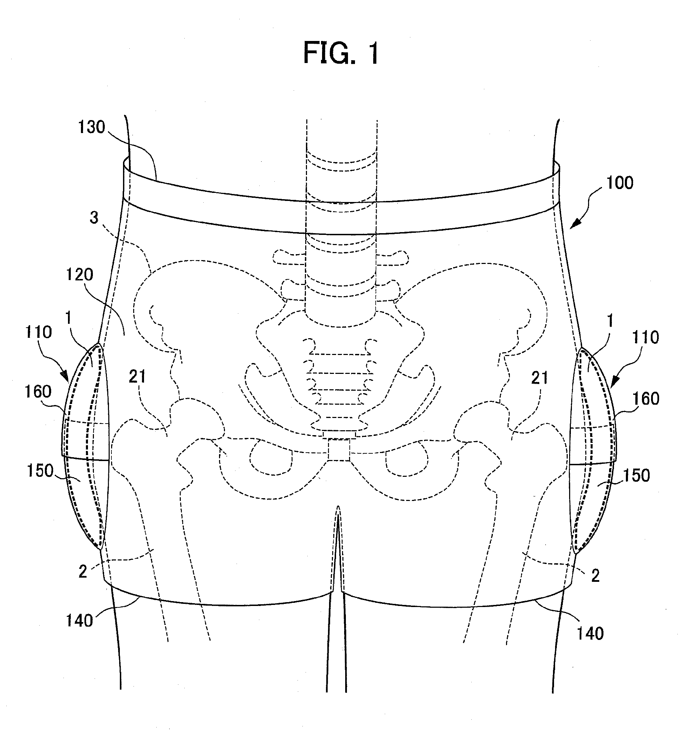

A protector 1 according to the present invention is mainly used to prevent a fracture of the femur occurring due to a fall by an elderly person. As illustrated in FIG. 1, the protector 1 is used while being positioned in a pair configuration on the right and the left at a position that covers the proximate region to the connecting portion of the femur 2 and the pelvis 3 of a wearer from the lateral orientation of the wearer. More particularly, the protector 1 is disposed to cover the side portion of the femoral-neck portion 21 that is the end portion on the side with the pelvis 3 in the femur 2.

The femoral-neck portion 21 is a position that tends to facture when an elderly person falls, and time is required to recover from a fracture after a fracture to the femoral-neck portion 21. When an elderly person suffers a fracture to the femoral-neck portion 21, it may be the case that a complete recovery is not possible. Since a long period in which walking is not possible is required to recover from a fracture, the muscular strength in the legs is adversely affected. As a result, a person may be confined to bed. Therefore, prevention of a fracture to the femoral-neck portion 21 of an elderly person due to a fall is extremely important.

In the present embodiment, as illustrated in FIG. 1, the protector 1 is attached by accommodation in an undergarment 100 that includes a pair of protector-containing portions 110. The undergarment 100 will be described below.

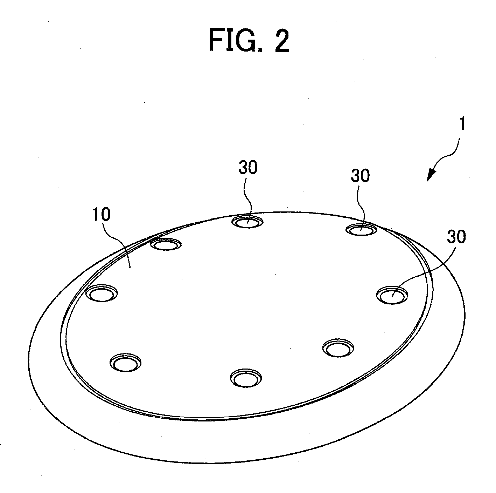

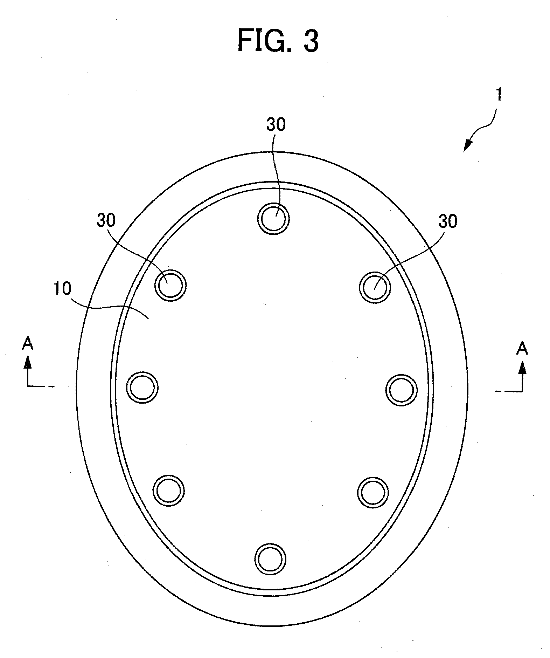

As illustrated in FIG. 2 to FIG. 4, the protector 1 according to the first embodiment includes a substantially circular shape in a plan view, and is curved in relation to the direction of thickness so that the outer surface is convex and the inner surface 20 is concave. As used herein, a substantially circular configuration is a concept that includes an ellipse or ovoid in addition to a circle.

A plurality of holes 30 is formed in the protector 1. The plurality of holes 30 is formed in proximity to the peripheral edge portion in the plan view of the protector 1 and pierces the protector 1 in relation to the direction of thickness. The plurality of holes 30 is respectively formed with a predetermined interval in the peripheral direction of the protector 1 at a position separated by a substantially equal distance from the peripheral edge. In the first embodiment, a total of eight holes 30 are formed at equal intervals in the peripheral direction of the protector 1.

The shock absorbing capacity can be improved when a force is applied to a position removed from the central portion of the outer surface 10 of the protector 1 (for example, a peripheral portion separated by 2 cm to 4 cm from the central portion) by forming of the plurality of holes 30 in proximity to the peripheral edge portion.

The respective diameter of the plurality of holes 30 is preferably 4 mm to 8 mm. The total of the surface area in the plan view of the plurality of holes 30 is preferably 1% to 10% of the surface area of the outer surface 10 when the protector 1 is deformed in a flat configuration. When the total of the surface area in the plan view of the plurality of the holes 30 is less than 1% of the surface area of the outer surface 10, the air permeability characteristics between the outer surface 10 and the inner surface 20 of the protector 1 through the plurality of holes 30 may not be sufficient. When the total of the surface area in the plan view of the plurality of the holes 30 exceeds 10% of the surface area of the outer surface 10, the protector 1 may not achieve a sufficient shock absorption capacity.

The size of the protector 1 in the plan view preferably has a long diameter of 135 to 180 mm and a short diameter of 110 mm to 155 mm in view of sufficient coverage of the femoral-neck portion 21 of the wearer.

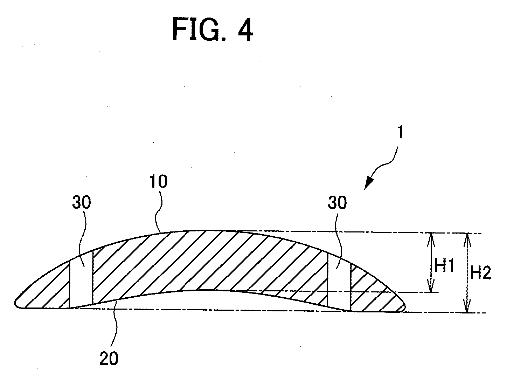

As illustrated in FIG. 4, the thickness of the protector 1 is approximately uniform in the portion excluding the peripheral edge portion. The thickness of the protector 1 gradually decreases continuously to the outer side in the peripheral edge portion. The thickness H1 in the portion excluding the peripheral edge portion of the protector 1 is preferably no more than 20 mm, and still more preferably 10 to 15 mm in view of not causing an unpleasant feeling to a wearer resulting from the thickness of the protector 1 when worn.

The thickness H2 from the flat surface to the top of the outer surface of the protector 1 when the protector 1 is disposed on the flat surface with the inner surface 20 below (hereinafter referred to as apparent thickness) is preferably 10 mm to 25mm. When the apparent thickness H2 of the protector 1 is less than 10 mm, the protector 1 may not exhibit sufficient shock absorbing capacity. When the apparent thickness H2 of the protector 1 exceeds 25 mm, the wearer may experience an unpleasant feeling as a result of the thickness of the protector 1 when worn.

The protector 1 includes a single-layer polymer-foam body, and exhibits shape restoring characteristics and flexibility. That is to say, the protector 1 can deform from a curved configuration to a substantially planar configuration (flat configuration) when a predetermined force is applied to the outer surface 10, and can return from the substantially planar configuration (flat configuration) to the curved configuration when the applied force is released.

The foam body that configures the protector 1 is enabled by use of a polyolefin resin such as polyethylene, an ethylene vinyl acetate copolymer (EVA), an ethylene-methyl acrylate copolymer (EMA), an ethylene ionomer, polypropylene, a polypropylene copolymer, and the like, a polyurethane resin, a polystyrene resin, and a polyvinyl chloride resin. Of the examples of resins given above, a foam body of EVA resin is preferred for use as a foam body from the point of view of high durability and high resilience.

The density of the polymer-foam body that configures the protector 1 is preferably 0.60 to 1.50 g/cm³, and more preferably 0.8 - 1.10 g/cm³. When the density of the polymer-foam body is less than 0.60 g/cm³, the protector 1 may not exhibit sufficient shock absorbing capacity. When the density of the polymer-foam body is more than 1.50 g/cm³, the protector 1 may not be sufficiently flexible and the wearer may experience an unpleasant feeling resulting from the hardness of the protector 1.

The protector 1 described above exhibits compressive characteristics in that the displacement amount in the thickness direction is 3 mm to 20 mm when a 20N force is applied to a central portion of the outer surface 10 in a state in which the protector 1 is disposed on a flat surface. When the displacement amount in the thickness direction of the protector 1 is less than 3 mm when a 20N force is applied to a central portion of the outer surface 10, the protector 1 may not be sufficiently flexible. When the displacement amount in the thickness direction of the protector 1 is more than 20 mm when a 20N force is applied to a central portion of the outer surface 10, the apparent thickness H2 of the protector 1 may become overly large, and an unpleasant feeling may result due to the thickness of the protector 1 when worn.

It is preferred that the protector 1 has a displacement amount of at least 10 mm when a force of 6N is applied to the protector 1 from the transverse direction or the longitudinal direction that is the direction which is orthogonal to the thickness direction. When the displacement amount is less than 10 mm when a force of 6N is applied from the transverse direction or the longitudinal direction of the protector 1, that is to say, when a force exceeding 6N is required for a 10 mm displacement, the tracking characteristics in relation to a bodily movement of a wearer may be adversely affected and the wearer may have an unpleasant feeling.

The protector 1 exhibits a shock absorbing capacity that enables a reduction in a force of 9200 N applied in a thickness direction from the outer surface side to less than or equal to 3000 N on the inner surface side.

Generally, the force applied to the femur during a fall is of the level of 5600 N to 8600 N. When a force is applied to the femur of an aged person (for example a female of 65 years of age or more), there is a high probability of a fracture when the applied force is more than 3000 N. That is to say, the protector 1 according to the present embodiment reduces the intensity of the shock applied to the femur during a fall of an aged person wearing the protector 1 to less than the intensity at which there is a high risk of a fracture. Consequently, the effect of preventing a fracture to the femur when an aged person falls can be enhanced.

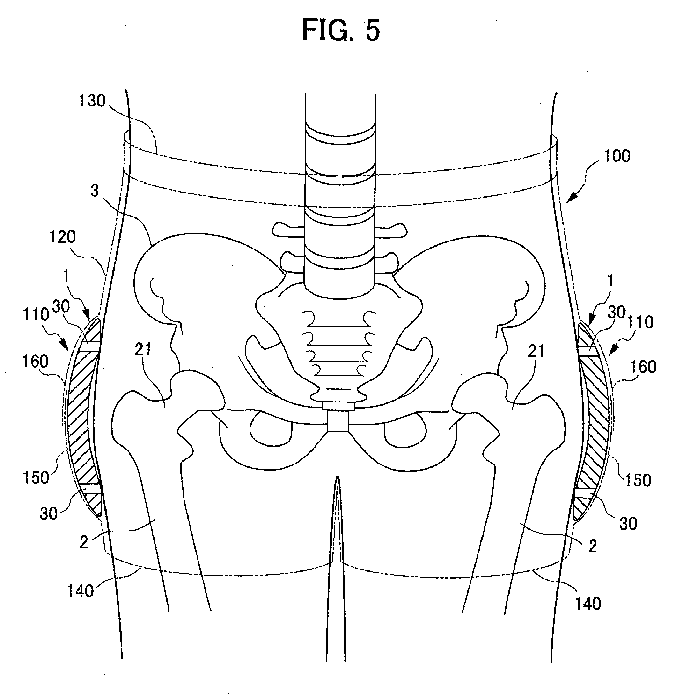

Next, the behavior of the protector 1 when a predetermined force is applied to the protector 1 will be described with reference to a state in which the protector 1 according to the present embodiment is worn. FIG. 5 is a sectional view illustrating a wearer who wears the protector 1.

As illustrated in FIG. 5, in a state in which the protector 1 is disposed so that the lateral orientation of the femoral-neck portion 21 of a wearer is covered, since the inner surface 20 of the protector 1 is formed with a concave shape, the predetermined space is formed between the inner surface 20 of the protector 1 and the skin side of the wearer.

In this state, for example, when the wearer makes an action to bend the hips, a bending force is applied onto the protector 1 in a longitudinal direction. In this context, the protector 1 is configured so that the displacement amount is at least 10 mm when a force of 6N is applied in a longitudinal direction. In this manner, the protector 1 can deform in response to an action to bend the hips and track (follow) the bodily movement of the wearer. In this manner, when the wearer performs an action to twist the body, although a warping force is applied in a transverse direction to the protector 1, the protector 1 is configured so that the displacement amount when a 6N force is applied in a transverse direction is at least 10 mm. In this manner, the protector 1 can deform in response to an action to twist the body and track the bodily movement of the wearer. In this manner, the protector 1 can deform even in response to a weak force such as during an action to twist the body or an action to bend the hips and thereby track the bodily movement of the wearer.

In a configuration when the protector 1 is worn, for example, a weak force from the outer surface 10 is applied to the protector 1 when making contact with another person. In this context, the protector 1 is configured so that the displacement amount in a thickness direction is 3 mm to 20 mm when a 20N force is applied to the central portion of the outer surface 10. In this manner, the protector 1 can deform even in relation to a weak force such as when contact is made with another person. In that situation, the air in the space between the inner surface 20 of the protector 1 and the skin of the wearer is guided into an external portion through the plurality of holes 30. As a result, the deformation to a flat configuration by the protector 1 is not impeded.

Consequently, the protector 1 can easily track a bodily movement during the everyday life of the wearer.

On the other hand, when the wearer who is wearing the protector 1 falls, a strong force (for example, a force of the order of 9000 M) is applied from the outer surface 10 to the protector 1. In response, the protector 1 exhibits a shock absorbing capacity to reduce a force of 9200 N applied in the thickness direction from the outer surface 10 to less than or equal to 3000 N on the inner side. In this manner, the force applied to the body of the wearer (the femoral-neck portion 21) as a result of the fall can be reduced to a level (less than or equal to a level of 3000N) at which an aged person will not suffer a fracture.

In this situation, the protector 1 receives the application of a force when deformed to a flat configuration, and thereby is subjected to a shock from a fall by use of substantially the whole surface of the inner surface 20 of the protector 1. In this manner, since the application of a force due to fall is dispersed on the whole surface of the inner surface 20 of the protector 1, the occurrence of a fracture resulting from concentration of the force applied to the protector 1 to a specific portion of the body of the wearer can be prevented.

Next, shorts 100 as a first embodiment of the undergarment according to the present invention that contains the protector 1 will be described making reference to FIG. 1 and FIG. 5.

The shorts 100 are configured by sewing of a cotton including material that exhibits superior flexibility and superior compressing characteristics. As illustrated in FIG. 1 and FIG. 5, the shorts 100 include a hip encircling portion 120, a waist opening portion 130, a pair of leg opening portions 140, and a pair of protector containing portions 110.

The hip surrounding portion 120 covers and encircles the hips including the peripheral portion in proximity to the femoral-neck portion 21 of the wearer. The waist opening portion 130 is provided on one end of the hip surrounding portion 120. The pair of leg opening portions 140 is provided on the other end of the hip surrounding portion 120. The pair of leg opening portions 140 respective allows passage of the legs of the wearer.

The pair of protector containing portions 110 is provided at a position corresponding to both side portions of the femoral-neck portion 21 of the wearer in the hip surrounding portion 120. In the present embodiment, the protector containing portions 110 are configured from a pocket portion 150 provided on the outer surface of the hip surrounding portion 120 and with an open upper end, and a covering portion 160 to cover the opening of the pocket portion 150.

The pocket portion 150 has a size that enables containment of two thirds of the length of the protector 1 in the longitudinal direction. The covering portion 160 has a size that enables covering of one half of the length of the protector 1 in the longitudinal direction. That is to say, in a state in which the protector 1 is contained in the protector containing portion 110, the overall surface of the outer surface 100 of the protector 1 is covered by the pocket portion 150 and the covering portion 160. In this state, the covering portion 160 includes a portion that overlaps with the pocket portion 150, and in the overlapping portion, the covering portion 160 is disposed on an outer side of the pocket portion 150. The pocket portion 150 and the covering portion 160 are both configured by sewing a fabric member on the outer surface side of the hip surrounding portion 120.

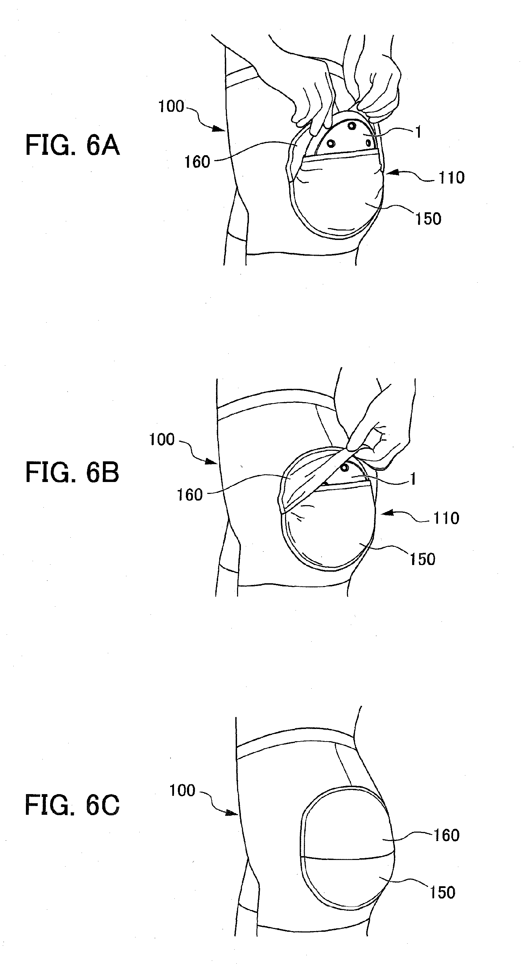

Next, an example of a wearing method of the protector 1 according to the present invention will be described making reference to FIG. 6A to 6C. FIG. 6A to 6C illustrate the sequence of wearing the protector 1.

Firstly, the wearer puts on the shorts 100 that include the protector containing portion 110. Then, as illustrated in FIG. 6A, the covering portion 160 of the protector containing portion 110 of one of the protector containing portions 110 is raised, and thereby the protector 1 is inserted and contained in the pocket portion 150. When the protector 1 is contained in the pocket portion 150, then, as illustrated in FIG. 6B and 6C, the covering portion 160 is applied on the outer side of the pocket portion 150. Then, the protector 1 is contained in the same manner in the other protector containing portion 110. In this manner, the protector 1 is contained simply in the protector containing portion 110 and disposed at a position at which the side portion of the femoral-neck portion 21 of the wearer is covered.

The protector 1 in the embodiment as described above obtains the following effect.

The protector 1 has a single-layer structure. In this manner, a configuration is possible in which the thickness of the protector 1 is reduced. The displacement amount in the thickness direction is configured as 3mm to 20 mm when a 20N force is applied from the outer surface 10 in the thickness direction of the protector 1. In this manner, the shock absorbing characteristics can be ensured and the tracking characteristics in response to the bodily movement of the wearer can be maintained. Therefore, a shock on the body of the wearer when the wearer falls can be reduced and an unpleasant feeling given to the wearer during long-term wearing can be reduced.

Furthermore, when the protector 1 is worn, the protector 1 is curved so that a space is formed between the skin side of the wearer and the inner surface 20 of the protector 1, and a flexibility is obtained that enables deformation to a flat configuration when a force of 20N is applied to the protector 1 from the outer surface 10 in the thickness direction. In this manner, when a weak force is applied to the protector 1 due to a bodily movement of the wearer, the space to the skin side of the wearer is utilized to facilitate deformation of the protector 1 into a flat configuration. On the other hand, when a strong force is applied to the protector 1, a shock can be reduced by the protector 1 that has deformed into a flat configuration. Therefore, the shock applied to the body of the wearer can be reduced when the wearer falls, and an unpleasant feeling on the wearer can be reduced during long-term wearing.

When a protector is configured in a double-layer structure of an outer layer and an inner layer, the outer layer and the inner layer may become separated during long-term use of the protector. In the present embodiment, since the protector 1 has a single-layer structure, there is no separation even during long-term use of the protector 1. Furthermore, the manufacturing process of the protector 1 is simplified since the protector can be manufactured without a step of bonding of the outer layer and the inner layer.

Generally, a force applied to the femur during a fall is of the order of 5600 to 8600 N. When a force is applied to the femur of an aged person (for example, a female of at least 65 years of age), the intensity of a force that causes a fracture is of the order of 3000 N. Therefore, shock absorbing characteristics can be ensured in the protector 1 to reduce the force of 9200N applied from the outer surface 10 of the protector 1 to less than or equal to 3000 N on the inner surface. In this manner, the intensity of the shock applied to the femur during a fall by an aged person wearing the protector 1 can be reduced to less than the applied intensity associated with a high risk of fracture. Therefore the effect of preventing fracture of the femur when an aged person falls can be enhanced.

Furthermore, the displacement amount when a force of 6N is applied from the longitudinal or the width direction of the protector 1 is configured to be at least 10 mm. In this manner, since deformation of the protector 1 is facilitated in the longitudinal and transverse directions, tracking characteristics can be further enhanced in relation to the bodily movements of the wearer. Therefore, an unpleasant feeling given to the wearer can be further reduced during long-term wearing.

The density of the polymer-foam body is 0.6 to 1.50 g/cm³. In this manner, an unpleasant feeling given to the wearer can be further reduced during long-term wearing and suitable shock absorbing characteristics can be maintained.

Furthermore, the protector 1 exhibits shape restoring characteristics. In this manner, since the protector 1 returns to a curved shape when the applied force is released, an unpleasant feeling given to a wearer can be reduced.

Furthermore, the protector 1 is configured by including of a plurality of holes 30 that pierce the thickness direction. In this manner, air in the space between the inner surface 20 of the protector 1 and the skin side of the wearer can interflow with the external air through the plurality of holes 30. In other words, when a weak force is applied to the protector 1, and the protector 1 deforms into a flat shape, the air in the space between the protector 1 and the wearer is guided into the external portion, and when the shape of the protector 1 returns to the curved shape, the air in the external portion is guided into the space. Therefore, since air suitably flows between the external portion and the space between the protector 1 and the wearer, comfort when the protector 1 is worn can be enhanced.

Furthermore, the plurality of holes 30 is provided in proximity to the peripheral edge portion of the protector 1. In this manner, when the force is applied to the outer surface 10 of the protector 1, polymer-foam body forming the protector 1 moves into the space of the holes 30 to deform largely (increase in flexibility). Therefore, the shock can be more effectively reduced in comparison to not providing the plurality of holes 30.

The apparent thickness of the protector 1 is configured as 10 mm to 25 mm. In this manner, an unpleasant feeling resulting from the thickness of the protector 1 given to the wearer can be reduced.

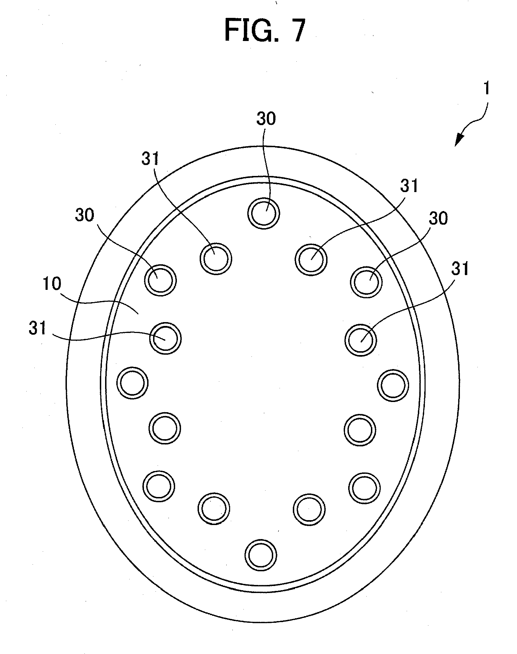

Next, a second embodiment of a protector according to the present invention will be described with reference to FIG. 7. FIG. 7 is a plan view of the protector 1 according to the second embodiment. Those constituent elements in the description of the second embodiment that are the same are denoted by the same reference numerals and description will be simplified by omitting repetitive description.

The protector 1 according to the second embodiment differs from the first embodiment in relation to the disposition of the plurality of holes 30. More specifically, the protector 1A according to the second embodiment includes a plurality of first holes 30 provided in proximity to the peripheral edge portion and a plurality of second holes 31 provided more on the inner side than the first holes 30.

The plurality of first holes 30 includes a total of eight holes in the same position as the first embodiment. The plurality of second holes 31 is provided respectively at approximately intermediate positions of two first holes 30. That is to say, a total of eight second holes 31 is provided. In this manner, a total of sixteen holes being first holes 30 and second holes 31 is provided in the protector 1 according to the second embodiment.

The protector 1 according to the second embodiment obtains the following effects in addition to the same effect as the first embodiment.

The plurality of holes include the plurality of first holes 30 and the plurality of second holes 31 that is provided more on the inner side of the plurality of first holes 30. In this manner, the shock absorbing capacity when a force is applied to a position separated from the central portion of the outer surface 10 of the protector 1 (for example, a peripheral portion separated by 2 cm to 4 cm from the central portion) can be further enhanced. Furthermore, the difference can be reduced between the shock absorbing capacity in the central portion of the outer surface 10 of the protector 1 and the shock absorbing capacity in the peripheral portion of the protector 1.

### EXAMPLES

Next, the present invention will be described in detail by use of examples and comparative examples. However, the present invention is not limited in any manner to the examples.

The following measurements were performed using a protector according to the first example and the second example in addition to the first comparative example to the fourth comparative examples.

The protector according to the first example has a single-layer structure configured from an EVA foam body having a density of 0.95 g/cm³ as a polymer-foam body. The protector according to the first example has a length along a long axis of 152 mm, a length along a short axis of 127 mm, a thickness in a central portion of 12 mm and an apparent thickness in a central portion of 22 mm. The protector according to the first example includes a total of eight holes provided at an equal interval in a peripheral direction in proximity to the peripheral edge portion.

The protector according to the second example uses the same configuration as the first embodiment apart from the different number of holes. The protector according to the second example includes a total of eight first holes provided at an equal interval in the peripheral direction in proximity to the peripheral edge portion and a total of eight second holes provided at an equal interval in the peripheral direction more on the inner side than the first holes. The plurality of second holes is respectively provided at approximately an intermediate position to the two first holes.

A first comparative example of a protector employs non-foam ethylene resin bonded by thermal fusion to the outer surface of the protector according to the first example. The protector according to the third comparative example has a length along a long axis of 152 mm, a length along a short axis of 122 mm, a thickness in a central portion of 23 mm and an apparent thickness in a central portion of 33 mm.

The protector according to the second comparative example includes a reinforcing core formed from a plate member of hard polypropylene (manufactured by Tytex A/S) and a polypropylene foam body covering the reinforcing core (manufactured by Tytex A/S). The protector according to the first comparative example has a length along a long axis of 157 mm, a length along a short axis of 112 mm, a thickness in a central portion of 8 mm and an apparent thickness in a central portion of 20 mm.

The protector according to the third comparative example has a single-layer structure formed from a polypropylene foam body having a density of 0.3 g/cm³. The protector according to the third comparative example has a length along a long axis of 150 mm, a length along a short axis of 120 mm, a thickness in a central portion of 12 mm and an apparent thickness in a central portion of 12 mm.

The protector according to the fourth comparative example has a double layered structure in which a polyurethane foam body having a density of 0.3 g/cm³ and a thickness of 5 mm is bonded using an adhesive onto the inner surface of the protector according to the first example. The protector according to the fourth comparative example has a length along a long axis of 152 mm, a length along a short axis of 122 mm, a thickness in a central portion of 17 mm and an apparent thickness in a central portion of 27 mm.

### Measurement of the Displacement Characteristics in the Thickness Direction

The relationship was measured between the displacement amount in the thickness direction, and the intensity of the load when a load is applied on a central portion of the outer surface protector according to the first example and the first to the fourth comparative examples.

The measurement of the displacement amount was performed in compression measurement mode using an "Autograph" manufactured by the Shimadzu Corporation.

The protector is mounted on the flat plate with the inner surface in a downward orientation in the protector according to the first example and the first to the fourth comparative examples. The central portion of the outer surface of the protector disposed on the flat plate is compressed from an upward to downward at a rate of 1mm/min on a 50N load cell (20 mm diameter contact surface with the protector) to thereby obtain a compression curve. The results are shown in Table 1.

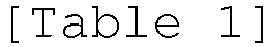

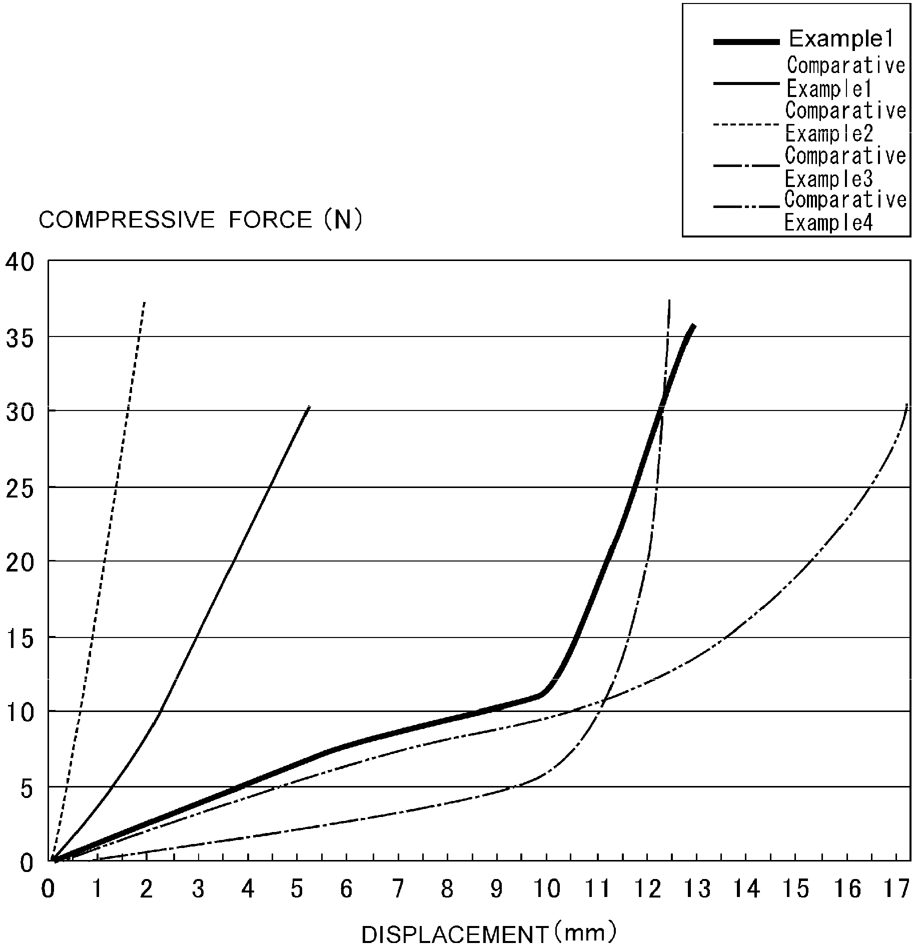

As shown in Table 1, the protector according to the first example requires a compressive force of 11N to obtain a 10 mm displacement (compression), and exhibits superior flexibility in comparison to the first comparative example and the second comparative example.

The protector 1 according to the first example exhibits two-stage compressive characteristics when a force is applied from the thickness direction to the central portion of the outer surface 10. In other words, the compression curve from a state in which the displacement amount exceeds 10 mm exhibits a sharp incline compared to the gentle compression curve for the displacement amount up to 10 mm. This illustrates the feature that the protector according to the first example facilitates deformation as a result of a comparatively weak force from a curved configuration to a flat configuration. Measurement of the Displacement Characteristics in the Longitudinal Direction

The relationship was measured between the displacement amount in the longitudinal direction and the intensity of the load when applying a load in the longitudinal direction in a protector according to the first example and the first and second comparative examples.

The measurement of the displacement amount was performed in compression measurement mode using an "Autograph" manufactured by the Shimadzu Corporation.

One end in the longitudinal direction of the protector is compressed from upward to downward at a rate of 1 mm/min on a 50N load cell (diameter 75 mm) in a state in which the protector is standing on a flat plate so that the longitudinal direction moves along the vertical direction to thereby obtain a compression curve in relation a protector according to the first example and the first and the second comparative examples.

The results are shown in Table 2.

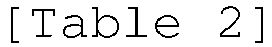

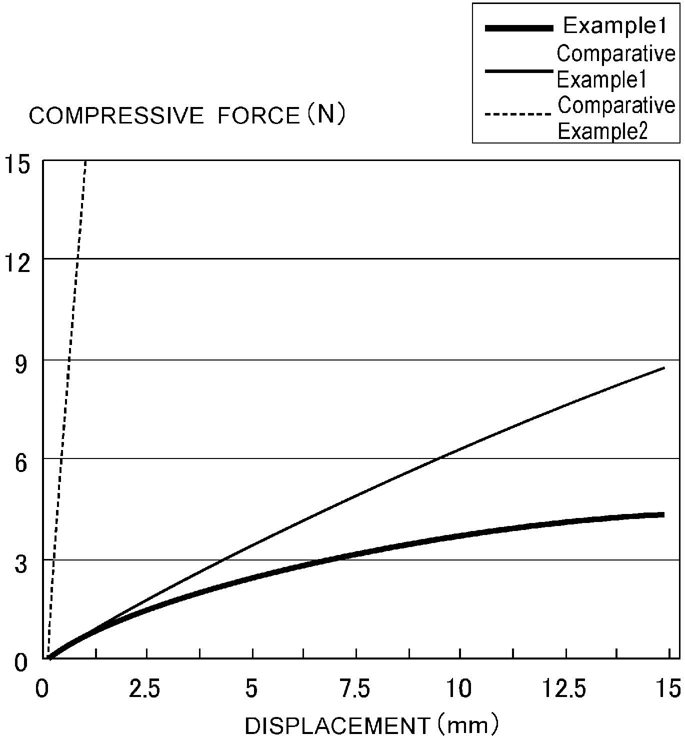

As shown in Table 2, the protector according to the first example exhibits large displacement characteristics in response to a small compressive force when compared with the protector according to the first comparative example and the second comparative example. This illustrates the feature that the protector according to the first example exhibits superior flexibility (pliancy) in the longitudinal direction compared to the protector according to the first comparative example and the second comparative example.

### Shock Absorbing Test 1

The shock absorbing capacity when a strong shock is applied from the thickness direction to a central portion is measured according to the protector according to the first example and the first to the third comparative example.

The measurement of the shock absorbing capacity is performed according to the following procedure.
(1) The protector is adhered using a double-sided tape to a semispherical body (corresponding to the buttocks of a human) having a weight of 4.7 kg and a diameter of 15 cm.
(2) The semispherical body with the protector attached is oriented to a measurement plate disposed 43 cm below, and the central portion of the protector is dropped freely to collide with the measurement plate. A pickup acceleration sensor manufactured by Tokushu Kiki K.K. is housed in the measurement plate.
(3) The shock when the semispherical body makes contact with the measurement plate was measured.
The results are shown in Table 3.

**[Table 3]**

| | No Protector | Example 1 | Comparative Example 1 | Comparative Example 2 | Comparative Example 3 |
|---|---|---|---|---|---|
| SHOCK (N) | 9214 | 2615 | 2134 | 3219 | 4900 |
| SHOCK ABSORBING RATE (%) | 0 | 71.6 | 76.8 | 65.1 | 46.8 |

As shown in Table 3, the protector in the first example exhibits a reduction in a 9212 N shock when in a blank configuration (configuration without a protector) to less than or equal to 3000. That is to say, in the protector according to the first example, the shock applied in the thickness direction can be reduced to less than or equal to one third, and the force applied to the femur during a fall (of the order of 5600 N to 8600 N) can be reduced to less than or equal to a force (of the order of 3000 N) at which there is a high risk of a fracture. On the other hand, the 9210 N shock is only reduced to 4900 N in the protector according to the third comparative example, and a sufficient reduction in the force applied to the femur during a fall is not enabled.

### Shock Absorbing Test 2

The shock absorbing capacity was measured when a strong shock is applied respectively to the central portion and the peripheral portion on the outer surface of the protector according to the protector according to the first example and the second example.

The measurement of the shock adsorbing capacity was performed using the same procedure as above (Shock Absorbing Test 1). However, in the procedure (2), the protector 1 was dropped in a free-falling manner such that the central portion of the protector, the position separated by 2 cm in the longitudinal direction from the central portion of the protector, a position separated 4 cm in the longitudinal direction from the central portion of the protector, a position separated 2 cm in the transverse direction from the central portion of the protector, and a position separated 4 cm in the transverse direction from the central portion of the protector were to collide with the measurement plate.

The results are shown in Table 4.

**[Table 4]**

| (UNIT: N) | | |
|---|---|---|
| Measurement Point | Example 1 | Example 2 |
| Central Portion | 2597 | 2724 |
| Longitudinal Direction 2 cm | 2778 | 2739 |
| Longitudinal Direction 4 cm | 3200 | 3009 |
| Transverse Direction 2 cm | 3435 | 3092 |
| Transverse Direction 4 cm | 4057 | 3631 |

As shown in Table 4, the shock absorbing capacity in particular of the central portion is superior in a protector according to the first example that includes eight holes. Furthermore, the protector according to the second example provided with eight first holes and eight second holes exhibits a small difference between the shock absorbing capacity in the central portion and the shock absorbing capacity in the peripheral portion, and enables suitable absorption of a shock on the whole surface of the protector.

### Measurement of Unpleasant Feeling When Worn

In order to confirm the unpleasant feeling reduction effect, a use test was performed in relation to the protector in the first example and the first comparative example.

The proportion of continuous use during five-days wearing was measured by use of a test subject in a state in which the test subject wears the shorts including a protector containing portion in which the protector is contained. After wearing, an interview-based survey was conducted in relation to whether or not there was an unpleasant feeling due to wearing.

The results are shown in Table 5.

**[Table 5]**

| | Example 1 | Comparative Example 1 |
|---|---|---|
| Rate of Continuous Use (%) | 58 | 45 |
| Rate of Suspension Use (%) | 42 | 55 |
| Proportion Without Unpleasant Feeling (%) | 30 | 14 |
| Number of People | 33 | 29 |

As illustrated in FIG. 5, the protector according to the first example exhibits a high rate of continuous use and a low rate of suspension of use in comparison to the protector according to the first comparative example. The protector according to the first example exhibits a high proportion of test subjects that did not experience an unpleasant feeling in comparison to the protector according to the first comparative example. In other words, the protector according to the first example exhibits a reduction in the unpleasant feeling given to a wearer during long-term wearing.

Although the preferred embodiments of the present invention have been described, the present invention is not limited to the above embodiments.

For example, in the present embodiment, although the protector 1 is disposed to cover the lateral orientation of the femoral-neck portion 21 of the wearer by containing in shorts 100 having the protector containing portion 110, the present invention is not limited in this regard. In other words, the protector may be configured as an undergarment including a protector in which the protector is integrated with the shorts at a position corresponding to both side portions of the femoral-neck portion in the shorts, or the protector may be mounted at a position corresponding to both side portions of the femoral-neck portion in the shorts by use of a mechanical fastener or the like.

In the present embodiment, although the shorts 100 are configured by sewing of a cotton material, the present invention is not limited in this regard. In other words, the shorts may be configured by inclusion of elastomer fibers, or by knitting of a tricot in the direction of knitting.

### EXPLANATION OF REFERENCE NUMERALS

- 1: PROTECTOR
- 2: FEMUR
- 3: PELVIS
- 10: OUTER SURFACE
- 20: INNER SURFACE
- 30: HOLE
- 100: SHORTS (UNDERGARMENT)
- 110: PROTECTOR CONTAINING PORTION
- 120: HIP SURROUNDING PORTION
- 130: WAIST OPENING PORTION
- 140: LEG OPENING PORTION

## Claims

1. A protector having a substantially circular shape in a plan view, the protector being disposed at a position at which a region proximate to a femoral-neck portion is covered from the lateral orientation of a wearer; wherein the protector has a single-layer structure configured from a polymer-foam body; and
curves in a thickness direction so that an outer surface is convex and an inner surface is concave; and
a displacement amount in a thickness direction when a 20 N force is applied to a central portion of the outer surface when disposed on a flat surface is 3 mm to 20 mm.

2. A protector having a substantially circular shape in a plan view, the protector being disposed at a position at which a region proximate to a femoral-neck portion is covered from the lateral orientation of a wearer; wherein the protector has a single-layer structure configured from a polymer-foam body;
curves in a thickness direction so that an outer surface is convex and an inner surface is concave to thereby form a space between the inner surface and the skin side of a wearer when worn; and
deforms into a flat configuration when a 20 N force is applied to a central portion of the outer surface.

3. The protector according to claim 1 or 2, wherein a force of 9200 N applied from the outer surface in a thickness direction is reduced to less than or equal to 3000 N on the inner surface side.

4. The protector according to any one of claims 1 to 3, wherein a displacement amount in a direction that is orthogonal to the thickness direction when a 6 N force is applied from a direction orthogonal to a thickness direction is at least 10 mm.

5. The protector according to any one of claims 1 to 4, wherein the density of the polymer-foam body is 0.60 to 1.50 g/cm³.

6. The protector according to any one of claims 1 to 5, wherein the protector is configured with shape restoring characteristics when an applied force is released.

7. The protector according to any one of claims 1 to 6, further comprising a plurality of holes formed in proximity to the peripheral edge portion in the plan view, piercing in the thickness direction thereof.

8. The protector according to any one of claims 1 to 7, wherein an apparent thickness is 10 mm to 25 mm.

9. An undergarment configured to contain the protector according to any one of claims 1 to 8, the undergarment comprising:
a hip surrounding portion configured to cover a circumference of a region proximate to the femoral-neck portion of the wearer;
a waist opening portion provided on one end of the hip surrounding portion;
a pair of leg opening portions provided on the other end of the hip surrounding portion; and
a pair of protector containing portions provided at a position corresponding to both side portions in proximity to the femoral-neck portion of the wearer in the hip surrounding portion.

10. An undergarment comprising: a hip surrounding portion configured to cover a circumference of a region proximate to the femoral-neck portion of the wearer;
a waist opening portion provided on one end of the hip surrounding portion; and
a pair of leg opening portions provided on the other end of the hip surrounding portion; wherein
a protector according to any one of claims 1 to 8 is disposed at a position corresponding to both side portions in proximity to the femoral-neck portion that connects the pelvis and the femur of a wearer in the hip surrounding portion.
